# EUROPEAN PATENT APPLICATION

(11) **EP 4 529 946 A1**
(43) Date of publication of application: **02.04.2025**
(21) Application number: 23200596.7
(22) Date of filing: 28.09.2023
(51) Int. Cl.: A61N 1/375, H05K 3/00

(54) **ENERGY-REDUCED AND AUTOMATABLE JOINING BY MEANS OF NANOSTRUCTURES FOR CONTACTING ELECTRICAL AND MECHANICAL COMPONENTS OF ACTIVE AND MONITORING IMPLANTS**

(71) Applicant: BIOTRONIK SE & Co. KG, 12359 Berlin (DE)
(72) Inventor: Kirchner, Tim, 12683 Berlin (DE); Binias, Sofia, 10587 Berlin (DE); Hauser, Markus, 15907 Lübben (DE)
(74) Representative: Biotronik Corporate Services SE

(57) **Abstract**

The present invention relates to an implantable device, comprising: a first component, and a second component, wherein the first and the second component are joined to one another via a connection comprising nanostructures.

## Description

The present invention relates to a method for connecting components of an implantable device as well as to an implantable device comprising a nanostructure-based connection, particularly an implantable pulse generator (IPG) or an implantable cardioverter defibrillator.

Particularly, a subject matter of the present invention is the manufacture of an electrical or an electromechanical connection of electronic components to each other, between printed circuit boards, or between an electronic component and a printed circuit board, or between outer wiring ribbons and other outer wiring components, like feedthrough pins. Other configurations are also conceivable.

Regarding implantable devices, providing proper connections that are mechanically stable and particularly allow conducting of an electric current through the connection, often requires soldering or laser welding the respective connection.

However, it is desirable to provide connections between components of an implantable device that can be efficiently established during a manufacturing process and achieve a reliable long-lasting connection between the components to be joined, which is particularly important for implants that remain for longer time spans in an implanted state in the respective patient, so that electrical/mechanical connections are not allowed to fail.

Publications EP3592697A1, EP3593102A1, EP3592696A1, EP3711462B1, DE102018122007A1, WO21185617A1, WO21185616A1, WO21185619A1, WO21185618A1, WO21259607A1, WO22012903A1 provide technical background to the generation of nanowire-based connections.

Based on the above, the problem to be solved by the present invention is to provide a method and an implantable device that achieves a reliable and efficient connection between components of the device that ideally also allows to conduct an electrical current between the components via the connection.

This problem is solved by a method having the features of claim 1 and by an implantable device having the features of claim 15. Preferred embodiments of these aspects of the present invention are stated in the corresponding dependent claims and are described below.

According to claim 1 a method for connecting a first component and a second component of an implantable device via a connection comprising nanostructures is disclosed, wherein the nanostructures are provided by means of one of:
- Deposition of nanostructures using Direct Laser Interference Patterning (DLIP),
- Laser Induced Periodic Surface Structures (LIPPS),
- 3D printing,
- laser cladding,
- ablation of a material,
- non-galvanic deposition of a material

The term "nanostructure" is used within its meaning known to skilled person. It particularly refers to a structure characterized by at least one dimension, preferable two dimensions in the nano scale, e.g., equal to or below 1 µm.

In one embodiment, the nanostructures are designed in form of nanowires, particularly characterized by a diameter below 1 µM, e.g. 0.1 nm to 100 nm. In one embodiment the nanostructures, particularly the nanowires extend form a surface of the first component and/ the second component, i.e. the nanowires comprise a first end attached the surface and free second end. In one embodiment, the free second end of the nanowires is designed in form of a hook, respectively. In one embodiment, the free second end comprises one or more branching point, from which two or more branches extend.

Thus, the present invention allows to substitute complex procedures such as laser welding by nanowire-based connections. Furthermore, the components to be joined may be preprocessed to obtain a nanostructure, particularly nanowire coating or surface so that making the connection may be accomplished by simply pressing the components onto one another, which corresponds to a significant simplification compared to a usual welding process (directly from z-axis assembly to joining process). Furthermore, using nanostructures such as nanowires provides an alternative material-locking connection, may be accomplished with a sufficient speed (depending on the process parameters) and yields a minimization of the process risk related to joining components of an implantable device. Furthermore, a corrosion of the joining surfaces may be prevented, and the nanostructure or nanowire-based connection is sensible for purely mechanical connections, such as mounting an antenna to a housing of the implantable device. Furthermore, the nanostructure or nanowire-based connection may also be advantageously applied to an internal structure of a pacemaker, for instance. Furthermore, apart from the required nanowire structure on the respective surface, no extra parts are typically needed to make the connection and the production costs are low with respect to known techniques such as soldering or laser welding. The necessary joining forces can be kept low enough so that no damage occurs to sensitive components. Furthermore, it is possible to bond planar surfaces as well as curved surfaces to one another. Adhesion to non-metallic surfaces is also conceivable. Further, a minimal size for manufacturing requirements/component design is merely limited by the requirement to hold down/press down connection partners for handling purposes and for forming the respective connection. Furthermore, advantageously, non-value-adding activities can be avoided through appropriate material selection, e.g., by applying nanowires directly to suitable surfaces without applying extra-capable metallization. The present invention enables high manufacturing tolerances and allows to conduct nanowiring at component level. Moreover, nanowiring as well as making nanostructure and nanowire-based connections can be automated.

According to a preferred embodiment of the method, the implantable device is an implantable medical device such as an implantable pulse generator (IPG) (e.g., of a cardiac pacemaker), an implantable cardioverter defibrillator (ICD), an implantable cardiac monitor (ICM). Other types of active implantable devices or components of an implantable device are as well suitable, as for instance a neurostimulator, a medication pump, a stimulation electrode, etc.

According to a preferred embodiment of the present invention, instead of utilizing a galvanic process, the nanostructures are created by either adding and/or removing material to/from the respective surface. Particularly, processes such as Deposition of nanostructures using Direct Laser Interference Patterning (DLIP), Laser Induced Periodic Surface Structures (LIPSS), 3D printing, laser cladding, ablation of a material, or non-galvanic deposition of a material are employed.

Direct laser interference patterning (DLIP) is a nanofabrication technique that utilizes the interference of multiple laser beams to create periodic patterns on the surface of a material. Particularly, DLIP is used to generate the respective pattern by letting multiple laser beams interfere with one another so as to create an intensity pattern that is projected onto a surface of the respective component. A base material forming the surface is melted or evaporated in areas of constructive interference of said laser beams, e.g. depending on the pulse length of the respective laser beam, while it remains essentially unaffected in areas of destructive interference. By appropriately selecting the number and arrangement of the beams in relation to each other the type of pattern und thus the resulting nanostructure can be selected. Particularly, the nanostructure can be formed as a plurality of periodically distributed protrusions. Particularly, DLIP offers several advantages. It allows for high precision and versatility in creating various surface patterns, including lines, grids, dots, or more complex structures. The pattern dimensions can range from micrometers to sub micrometer scales, depending on the wavelength and parameters of the laser used. DLIP can be applied to a wide range of materials, including metals, semiconductors, polymers, and ceramics.

Furthermore, Laser-Induced Periodic Surface Structures (LIPSS) or ripples, refer to the formation of periodic surface structures on materials induced by laser irradiation. LIPSS occurs when a material is exposed to laser pulses, resulting in the self-organization of nanoscale or microscale structures on the surface.

Particularly, the formation of LIPSS involves several key factors. The laser parameters play a crucial role in LIPSS formation. These parameters include laser wavelength, pulse duration, fluence (energy density), repetition rate, and polarization. The choice of laser parameters determines the characteristics of the resulting LIPSS, such as period, orientation, and morphology. Furthermore, the material's optical, thermal, and mechanical properties influence the LIPSS formation. For instance, the absorption coefficient, thermal conductivity, and melting or ablation threshold of the material affect the energy deposition and heat transfer processes during laser irradiation.

Particularly, due to interaction of the laser beam with the material surface, various physical processes occur that include absorption of laser energy, generation of heat, melting, vaporization, and re-solidification. The interaction dynamics lead to the formation of surface structures, particularly nanostructures. Two particular types of LIPSS are the so-called Low Spatial Frequency LIPSS (LSFL) and High Spatial Frequency LIPSS (HSFL). LSFL, also known as ripples or periodic grooves, exhibit a periodic modulation with relatively long spatial periods ranging from a few hundred nanometers to several micrometers. LSFLs are typically parallel or perpendicular to the laser polarization direction and are formed by a combination of surface plasmon polaritons, interference effects, and material redistribution processes, whereas HSFLs, also known as spikes or subwavelength ripples, have smaller spatial periods ranging from tens to a few hundred nanometers. They may be oriented perpendicular or oblique to the laser polarization. HSFL formation mechanisms involve nonlinear absorption, plasma generation, and the interference of surface or guided waves.

Further, regarding 3D printing, nanostructures may be provided using Two-Photon Polymerization (TPP), wherein ultrafast laser pulses are used to selectively polymerize a photosensitive resin at the nanoscale, wherein particularly the resin essentially consists of or comprises an electrically conductive poly. Particularly, the laser may be tightly focused on the resin, allowing for precise control of the polymerization process. By scanning the laser focus point through the resin, complex 3D nanostructures with feature sizes in the range of tens to hundreds of nanometers can be printed. Furthermore, 3D printing such as Stereolithography can be used for printing at the nanoscale, wherein photosensitive resins are selectively solidified layer by layer by means of a focused laser beam or other light sources so as to form the respective nanostructures. Furthermore, also Electron-Beam Lithography (EBL) can be used. Although not a traditional 3D printing technique, EBL can be used to generate nanostructures on the respective surface with high precision. It involves using a focused electron beam to selectively expose a resist material. Particularly, the electron beam changes the solubility of the resist material, enabling selective removal of either the exposed or non-exposed regions of the resist by immersing it in a suitable solvent which is denoted as developing. The developed resist may then be used as a template for subsequent processes such as etching or deposition to create 3D nanostructures.

Furthermore, in laser cladding, material for forming the respective nanostructure is deposited onto the respective surface by melting a metallic powder or wire provided on the surface to be coated by means of a laser.

According to a preferred embodiment of the method according to the present invention the respective nanostructure comprises a length in the range from 100nm to 100 µm, and/or a diameter in the range from 10 nm to 10 µm. Particularly, the length is a length in a direction normal to a surface on which the respective nanostructure is provided.

Further, according to a preferred embodiment of the method according to the present invention, the respective nanostructure is an elongated nanowire (the nanowire comprising a length in the range from 100nm to 100 µm, and/or a diameter in the range from 10 nm to equal to or below 1 µm). Particularly, the term diameter refers to a circular base, but the respective nanostructure/nanowire may also have an oval or polygonal base.

According to yet another preferred embodiment, the respective nanostructure comprises at least one branching point at which the nanostructure branches out from a base into at least two sections.

Furthermore, according to an embodiment of the method the respective nanostructure comprises a free end section forming a hook.

Thus, due to comprising branches and/or hook-shaped free ends, the respective nanostructure promotes entanglement with an opposing nanostructure to facilitate a connection between the opposing nanostructures.

According to a preferred embodiment of the method according to the present invention, the nanostructures or -wires are formed out of one of the following metals or comprise one of the following metals: copper (Cu), platinum (Pt), gold (Au), Iridium (Ir), Ruthenium (Ru), Zirconium (Zr), titanium (Ti), niobium (Nb), nickle (Ni), tungsten (W), tantalum (Ta), MNP35N, or an alloy thereof. Particularly, metallic nanostructures may be used in all embodiments described herein.

Furthermore, according to a preferred embodiment of the method, first nanostructures are provided on a surface of the first component and second nanostructures are provided on a surface of the second component, wherein the first and the second nanostructures are interlocked with each other to form said connection.

Preferably, the connection may be generated by pressing the two surfaces comprising said first and second nanostructures (e.g. nanowires) onto one another with a pressure in the range from 15 MPa to 200 MPa over a time period in the range from 0.06 s to 60 s. Preferably, a temperature of the surfaces to be joined is about 20°C (room temperature). This process is also known as "KlettWelding". Advantageously, the joining process may be conducted at room temperature. In contrast to a brazing or welding process, the components do not expand thermally, which ensures stress-free joining. In addition, the process may be automated very well by simple means, since the joining time is just 0.06 s to 60 s long. A shear strength of up to 20 MPa can be achieved with this process.

Particularly, the joining of the components is based on two different modes of action. On the one hand, the contact pressure causes the nanostructures or -wires to deform and form a kind of mesh. This is very similar to the way a Velcro fastener works. On the other hand, the contact pressure causes a deformation of the nanostructures or nanowires in the area of the bonding plane. The surfaces of the nanostructures or nanowires are thus spaced apart by an order of magnitude of the atomic distance, e.g. between 0.1 nm and 1 nm). Through space change processes as well as through cohesion and adhesion forces between the atoms, a stable bond is achieved. When additional thermal energy is added to the joining process yielding a temperature of the surfaces to be joined in the range from 170°C to 240°C, the process is also denoted as "Klettwelding+" process. Application of thermal energy to the joining process allows to reduce the pressure, which is now preferably in the range from 10MPa to 100MPa. The contact pressure may thus be reduced and particularly lies between 10 MPa and 100 MPa. The shear strength is significantly increased and is at a maximum of typically 65MPa. Despite the joining temperature of a maximum of 240 °C, the joint is significantly less stressed than comparable welding or soldering processes, since, for example, soft soldering (lead-free solder) is only possible at 221 °C and brazing at 450 °C. The application of thermal energy reduces the place change processes while cohesive and adhesion forces are further enhanced. The process thus may be classified as a diffusion welding process.

Furthermore, according to a preferred embodiment of the method, the nanostructures, preferably metallic nanostructures, are provided on a surface of the first component, wherein a surface of the second component is particularly devoid of the nanostructures, and wherein the nanostructures and the surface of the second component are bonded to one another to form said connection, or wherein nanostructures, preferably metallic nanostructures, are provided on a surface of the second component, wherein a surface of the first component is devoid of the nanostructures, and wherein the nanostructures and the surface of the first component are bonded to one another to form said connection.

Particularly, here, the connection may be generated by pressing the surface comprising the nanostructures onto the surface that is free of nanostructures with a pressure in the range from 10 MPa to 100 MPa over a time period in the range from 120 s to 300 s. Preferably, a temperature of the surfaces to be joined is in the range from about 170°C to 240°C. This process is also denoted as "KlettSintering" process. Here, two components are joined together with only one of the two surfaces having nanostructures provided thereon. After positioning, the two surfaces of the components are pressed together under pressure and temperature (as in "KlettWelding+"). The mode of action of the joining process is thus also based on the approach of the surface of the nanostructures to the surface of the opposing component, so that diffusion processes take place at the atomic level. Thus, "KlettSintering" may also be classified as a diffusion welding process. It is to be noted that the usual sintering is primarily used to produce layers or entire workpieces and not to join components as described herein.

Furthermore, according to a preferred embodiment of the implantable device, the connection comprises an intermediate film having a first side and a second side facing away from the first side, wherein the nanostructures, particularly metallic nanostructures, are provided on the first and the second side, wherein the nanostructures provided on the first side are bonded to the surface of the first component, and wherein the nanostructures provided on the second side are bonded to the surface of the second component.

Particularly, here, the connection may be generated by pressing the surfaces of the first and second component onto one another with the intermediate film arranged between said surfaces. The nanostructures protruding from the respective side of the intermediate film then bond to the opposing surface of the first or second component. Particularly, the surfaces are pressed onto one another with a pressure in the range from 10 MPa to 100 MPa over a time period in the range from 120 s to 300 s. Preferably, a temperature of the surfaces to be joined is in the range from about 170°C to 240°C. This process is also denoted as "KlettSintering+" process. Here, neither surface has to be provided with the nanostructures. Instead, a thin film is used which has the nanostructures provided on both sides by one of the procedures described herein. In a preferred embodiment of the present invention, the film is about 10 µm to 30 µm thick, preferably about 20 µm thick. The joining parameters are the same as for "KlettSintering". Thus, "KlettSintering+" may also be classified as a diffusion bonding process. One advantage of the above-described diffusion bonding processes compared to a normal diffusion bonding process is that in normal diffusion bonding, the two surfaces to be joined have to be prepared in advance. This includes, for example, polishing work to obtain a surface that is as smooth as possible. Only in this way can they be brought to the necessary distance so that the diffusion processes can take place. Through the use of nanostructures this preparation can be omitted, as the nanostructures are long enough to compensate for unevenness and to enable a two-dimensional connection.

Furthermore, according to a preferred embodiment of the method, the nanostructures, preferably metallic nanostructures are provided on a surface of the first component and/or on a surface of the second component so as to preferably generate an electrical contact between the surfaces, wherein the two opposing surfaces are connected to one another by an intermediate adhesive layer to form the connection.

Particularly, here, the connection can be generated by pressing the surfaces of the first and second component onto one another with the intermediate adhesive layer arranged between said surfaces. The nanostructures, particularly nanowires, protruding from one or two of the surfaces generate an electrical connection between the surfaces. Particularly, the surfaces are pressed onto one another with a pressure in the range from 1 MPa to 5 MPa over a time period in the range from 0.06 s to 300 s. Preferably, a temperature of the surfaces to be joined and of the adhesive layer is in the range from 20°C to 150°C.

This process is also known as "KlettGluing" process. Here, the mechanical bond between the surfaces is not created by the nanowires, but by the use of an adhesive. In this process, the nanowires only serve to create an electrical connection between the two surfaces. The joining parameters and the resulting shear strengths depend only on the adhesive.

Furthermore, according to a preferred embodiment of the method, the connection comprises an intermediate film having a first side and a second side facing away from the first side, wherein the first and the second side are each provided with the nanostructures, preferably metallic nanostructures, particularly according to one of the procedures described herein, wherein the nanostructures provided on the first side are bonded to the surface of the first component via an adhesive layer, and wherein the nanostructures provided on the second side are bonded to the surface of the second component via an adhesive layer.

Particularly, here, the connection may be generated by pressing the surfaces of the first and second component onto one another with the intermediate film arranged between said surfaces. The nanostructures protruding from the respective side of the intermediate film then bond to the opposing surface of the first or second component via the respective adhesive layer. Particularly, the surfaces are pressed onto one another with a pressure in the range from 10 MPa to 100 MPa over a time period in the range from 120 s to 300 s. Preferably, a temperature of the surfaces to be joined is in the range from about 170°C to 240°C.

In case an additional film is used, the process is also denoted as "KlettGlueing+" process. In the gluing process without an additional intermediate film, at least one of the surfaces of the two components / joining partners is provided with a coating of nanowires. In contrast "KlettGlueing+" uses an additional film with both nanowire coatings being bonded to the respective opposing surface by an adhesive. Due to the use of adhesives, both processes can also be classified as gluing processes.

Furthermore, according to a preferred embodiment of the method, the respective adhesive layer is formed by an anaerobic adhesive, preferably a dimethacrylate ester-based adhesive. Particularly, such anaerobic adhesives belong to the group of chemically curing adhesives and their setting only begins in the absence of oxygen and in the presence of metal ions. Due to this, such adhesives are often used for screw locking or shaft-hub connection (a suitable product that may be used in the framework of the present invention as an adhesive is Loctite 243 of Henkel adhesives).

According to yet another preferred embodiment of the method, the nanostructures are provided on the respective surface of the component or side of an intermediate film so as to form a plurality of spaced apart regions, each region comprising a plurality of nanostructures, wherein surface areas in between two neighboring regions are devoid of the nanostructures.

According to a preferred embodiment, the respective connection is an electrically conducting connection (and based on metallic or otherwise electrically conducting nanostructures). Since the connection also allows the transfer of mechanical forces from one component to the other component, this connection can also be denoted as an electromechanical connection.

Furthermore, according to a preferred embodiment of the method, the connection forms a liquid-tight sealing between the first and the second component so as to prevent penetrations of liquids through the sealing (e.g., into a housing of the implantable device).

Furthermore, according to an embodiment of the method, a negative contact of a welding protector for the implant can be connected to the implant.

In each of the following embodiments, the implantable device may be - among other implants - an implantable pulse generator (IPG) such as a cardiac pace maker, an implantable cardioverter defibrillator (ICD) or an implantable neurostimulator.

According to a preferred embodiment of the method, the first component is a housing of the implantable device, and the second component is an X-ray marker that is bonded to the housing via said connection (said connection thus serves for mounting an X-ray detection). Other members can be bonded in the same way to the housing,

In a further preferred embodiment of the method, the first component is a shell of a housing of the implantable device, particularly a half shell, wherein the second component is a shell of the housing, particularly a half shell, and wherein said connection closes the shells of the housing hermetically (i.e. the connection connects the two shells, particularly half shells, hermetically to one another. Thus, the invention achieves pressing housing to housing, e.g. as a substitute for a welded circumferential seam for the assembly of two housing halves of an implant. In this embodiment, nanostructures, particularly nanowires, are to be provided on the contact surfaces of the half shells so that they can be joined by assembly. Particularly, in the above, the shells can be titanium shells and the half shells can be titanium half shells. Furthermore, according to a preferred embodiment, said connection closes a battery cover of the implantable device hermetically.

In a further preferred embodiment of the method, the implantable device comprises an energy storage, e.g. a battery or a capacitor, particularly a high voltage capacitor, comprising an electrical feedthrough pin forming the first component and a circuit arranged on a substrate, particularly in form of a printed circuit board, forms the second component, wherein said connection connects the electrical feedthrough pin to the circuit, particularly to the surface of a contact member of the circuit.

Furthermore, according to a preferred embodiment of the method, the implantable device comprises a printed circuit board forming the first component, the surface of the first component forming an electrical contact of the printed circuit board, and wherein the second component is an electronic component (i.e., said connection electrically connects the electronic component to the printed circuit board).

According to a preferred embodiment of the method, the implantable device comprises a dump resistor forming the first component and a printed circuit board forming the second component. Particularly, the dump resistor comprises an electrical contact member comprising a surface that is connected via said connection to a surface of an electrical contact member of the printed circuit board. The other electrical contact member can be connected to another electrical contact member of the printed circuit board in the same fashion.

According to yet another preferred embodiment of the method, the implantable device comprises a housing comprising an electrical feedthrough comprising at least one pin (particularly nail head pin) for providing an electrical connection to a header connected to the housing, wherein particularly the header is configured for providing an electrical connection to at least one electrode lead, and wherein the at least one pin is electrically connected to an electronic module enclosed by the housing of the implantable device, and wherein the at least one pin forms the first component, and the second component is an electrically insulating sleeve (e.g., out of a ceramic or glass material) that surrounds the at least one pin and is connected to the housing, wherein particularly said connection connects a surface of a head of the pin to a surface of the sleeve.

In a further preferred embodiment of the method, the at least one pin is surrounded by an electrically insulating sleeve (e.g., out of a ceramic or glass material) that forms the first component, and the second component is formed by the housing, wherein particularly said connection connects a bottom surface of the sleeve to a circumferential surface of the housing.

In a further preferred embodiment of the method, the at least one pin forms the first component, and the second component forms part of the header, wherein particularly the second component is an external wiring arranged outside the housing (e.g., an electrically conducting band) for connecting the at least one pin to a component of the header.

In a further preferred embodiment of the method, the first component is formed by an external wiring connected to the at least one feedthrough pin, and the second component is formed by a header core of the header, wherein particularly the header core is one of: a socket, a two-pole socket, a four-pole socket, an eight-pole socket. Particularly, these sockets are configured to receive a plug of an electrode lead, respectively.

In a further preferred embodiment of the method, the first component is formed by a first header core of the header and the second component is formed by a second header core of the header. Particularly, the first and/or second header core is a socket.

In a further preferred embodiment of the method, the at least one pin forms the first component, and the second component is an interconnect device, particularly a 3D molded interconnect device (MID), that is particularly configured to connect the at least one pin to a header core.

In a further preferred embodiment of the method, the first component is an interconnect device, particularly a 3D molded interconnect device (MID), that is particularly configured to connect the at least one pin to a header core, and wherein the second component is a header core of the header.

According to a preferred embodiment, the at least one pin of the above-described electrical feedthrough comprises a head that is flush with an outside of the housing.

Furthermore, according to yet another preferred embodiment, the external wiring is a flexible connector comprising a flat flexible substrate and at least one conductive track arranged thereon.

Furthermore, according to a preferred embodiment, the first component may be a lug connected to a connector and the second component an electrical contact member (e.g. of a PCB) that is connected via said connection to the lug.

Furthermore, according to a preferred embodiment, the first component is a battery of the implantable device and the second component is a circuit of the implantable device.

Furthermore, according to a preferred embodiment, the first component is a battery of the implantable device and the second component is a circuit of the implantable device.

Furthermore, according to a preferred embodiment, the first component is an antenna of the implantable device and the second component is a housing of the implantable device.

Further, said nanostructure-based connection according to the present invention as claimed in claim 1 and 15 may also be used in other devices, for instance external devices, particularly as replacement for screw processes. Furthermore, electrodes can be connected to a supporting structure via said nanowire-based connection. The nanowire-based connection according to the present invention as described herein can also be used as an alternative for fractal coating, e.g., of electrical functional surfaces (e.g., ring electrode Pt/Ir, titanium housing of miniaturized implants such as leadless pacemaker).

According to a preferred embodiment, the nanowires are made out of one of the following metals: copper (Cu), platinum (Pt), gold (Au), Iridium (Ir), Ruthenium (Ru), Zirconium (Zr).

Furthermore, according to a preferred embodiment, said external wiring may comprise or be formed out of suitable metals such as titanium, niobium, platinum, iridium, platinum/iridium (e.g., for feedthrough pins, and electrically conducting bands) and stainless steel, particularly NiCoCrMo (particularly MP35N).

Furthermore, according to a preferred embodiment, electrically conducting bands, circuit substrates (e.g. PCB) and flexible connectors connected to feedthrough pins comprise or are formed out of niob (Nb).

Furthermore, the respective electrically conducting band connected to a circuit or tab may comprise nanowires, wherein the tab can comprise CuNi, and the band can comprise CuNi oder Ni. Particularly, a plate can be coated for CuNi tabs and cut afterwards.

Furthermore, 4-pole modules (4-pole connector sockets) may comprise NiCoCrMo (particularly MP35N). Here, the components are all located in the header of the implant, i.e. outside the titanium housing; the area is encapsulated with resin; the pacemaker electrodes are configured to be connected to the connector sockets, which conduct the electrical signals from ("sense") and to ("pace") the heart.

According to a further aspect of the present invention an implantable device produced with the method according to the present invention is disclosed.

Furthermore, according to yet another aspect of the present invention an implantable device is disclosed, the implantable device comprising
- a first component, and
- a second component,
wherein
the first and the second component are joined to one another via a connection comprising nanostructures provided by one of:
- Deposition of nanostructures using Direct Laser Interference Patterning (DLIP),
- Laser Induced Periodic Surface Structures (LIPSS),
- 3D printing,
- laser cladding,
- ablation of a material,
- non-galvanic deposition of a material.

This implantable device can comprise any of the features of the implantable device described in conjunction with the method of the present invention.

According to a preferred embodiment of the implantable device, the latter is an implantable medical device such as an implantable pulse generator (IPG) (e.g. of a cardiac pacemaker), an implantable cardioverter defibrillator (ICD), an implantable cardiac monitor (ICM). Other types of active implantable devices or components of an implantable device are as well suitable, as for instance a neurostimulator, a medication pump, a stimulation electrode, etc.

According to a preferred embodiment of the implantable device, the respective nanostructure comprises a length in the range from 100nm to 100 µm, and/or a diameter in the range from 10 nm to equal to or below 1 µm.

Preferably, according to an embodiment of the implantable device, the respective nanostructure is an elongated nanowire (the nanowire comprising a length in the range from 100nm to 100 µm, and/or a diameter in the range from 10 nm to equal to or below 1 µm).

Furthermore, according to a preferred embodiment of the implantable device, the respective nanostructure comprises at least one branching point at which the nanostructure branches out from a base into at least two sections.

Furthermore, according to a preferred embodiment of the implantable device, the respective nanostructure comprises a free end section forming a hook.

Furthermore, according to a preferred embodiment of the implantable device, the connection comprises a coating of a surface of the first component with first metallic nanowires and a coating of a surface of the second component with second metallic nanowires, wherein the first and the second metallic nanowires are interlocked with each other.

Further, according to a preferred embodiment of the implantable device, the nanostructures, preferably metallic nanostructures, are provided on a surface of the first component, wherein the nanostructures and the surface of the second component are bonded to one another, or wherein the nanostructures, preferably metallic nanostructures, are provided on a surface of the second component, wherein the nanostructures and the surface of the first component are bonded to one another.

According to yet a further preferred embodiment of the implantable device, the connection comprises an intermediate film having a first side and a second side facing away from the first side, wherein the first and the second side are each provided with nanostructures, preferably metallic nanostructures, wherein the nanostructures provided on the first side are bonded to a surface of the first component, and wherein the nanostructures provided on the second side are bonded to a surface of the second component.

According to another preferred embodiment of the implantable device, the nanostructures, preferably metallic nanostructures, are provided on a surface of the first component and/or on a surface of the second component, wherein the connection further comprises an intermediate adhesive layer for connecting the two opposing surfaces to one another.

According to a further embodiment of the implantable device, the connection comprises an intermediate film having a first side and a second side facing away from the first side, wherein the first and the second side are each provided with nanostructures, particularly metallic nanostructures, wherein the nanostructures provided on the first side are bonded to a surface of the first component via an adhesive layer, and wherein the nanostructures grown on the second side are bonded to a surface of the second component via an adhesive layer.

According to yet another embodiment of the implantable device, the respective adhesive layer is formed by an anaerobic adhesive, preferably a dimethacrylate ester-based adhesive.

According to yet another preferred embodiment of the implantable device, the nanostructures are provided on the respective surface or side so as to form a plurality of spaced apart regions, each region comprising a plurality of nanostructures, wherein particularly surface areas in between two neighboring regions are devoid of the nanostructures.

Particularly, in each of the following embodiments, the implantable device may be - among other implants (see above) - an implantable pulse generator (IPG) such as a cardiac pace maker, an implantable cardioverter defibrillator (ICD), or an implantable neurostimulator.

According to preferred embodiment of the implantable device,
- the first component is a housing of the implantable device, and wherein the second component is an X-ray marker that is bonded to the housing via said connection, or wherein
- the first component is a shell of a housing of the implantable device, particularly a half shell, and wherein the second component is a shell of the housing, particularly a half shell, wherein said connection closes the shells of the housing hermetically.

Further, according to a preferred embodiment of the implantable device, the implantable device comprises an energy storage, e.g. a battery or a capacitor, comprising an electrical feedthrough pin forming the first component and a circuit arranged on a substrate, the circuit forming the second component.

According to yet another preferred embodiment of the implantable device, the implantable device comprises a printed circuit board forming the first component, the surface of the first component forming an electrical contact of the printed circuit board, and wherein the second component is an electronic component.

Preferably, in another preferred embodiment, the implantable device comprises a dump resistor forming the first component and a printed circuit board forming the second component.

According to yet another preferred embodiment of the implantable device, the implantable device comprises a housing comprising an electrical feedthrough comprising at least one pin for providing an electrical connection to a header connected to the housing, wherein the at least one pin is electrically connected to an electronic module enclosed by the housing of the implantable device, and wherein
- the at least one pin forms the first component, and the second component is an electrically insulating sleeve that surrounds the at least one pin and is connected to the housing, or wherein
- the at least one pin is surrounded by an electrically insulating sleeve that forms the first component, and the second component is formed by the housing, or wherein
- the at least one pin forms the first component, and the second component is arranged in the header, wherein particularly the second component is an external wiring arranged outside the housing for connecting the at least one pin to a component of the header, or wherein
- the first component is formed by an external wiring connected to the at least one feedthrough pin, and the second component is formed by a header core of the header, wherein particularly the header core is one of: a socket, a two-pole socket, a four-pole socket, or wherein
- the first component is formed by a first header core of the header and the second component is formed by a second header core of the header, or wherein
- the at least one pin forms the first component, and the second component is an interconnect device, particularly a 3D molded interconnect device, that is particularly configured to connect the at least one pin to a header core, or wherein
- the first component is an interconnect device, particularly a 3D molded interconnect device, that is particularly configured to connect the at least one pin to a header core, and wherein the second component is a header core of the header.

In the following, preferred embodiments of the present invention as well as further features and advantages of the present invention are described with reference to the Figures, wherein
- Fig. 1: shows an embodiment of an implantable device according to the present invention, wherein an electrical feedthrough pin is connected via a nanostructure-based connection to a housing of the implantable device; particularly different configurations of said connection are shown,
- Fig. 2: shows a further embodiment of an implantable device according to the present invention, wherein an electrical feedthrough pin is connected via an electrically conducting band to a header core using nanostructure-based connections,
- Fig. 3: shows a further embodiment of an implantable device according to the present invention, wherein the header comprises an interconnect device, particularly an MID, that connects an electrical feedthrough to header cores of the header of the implantable device, wherein connections between feedthrough, interconnect and header cores can be nanostructure-based connections,
- Figs. 4A-4B: show a further embodiment of an implantable device according to the present invention, wherein an electrical feedthrough pin is connected via an electrically conducting band to a header core of a header of the implantable device,
- Figs. 5: show a modification of the embodiment of Fig. 4, wherein here a flexible connector is used to connect feedthrough pins to a curved header core,
- Figs. 6A-6B: show external wiring of two-pole header cores connected to feedthrough via a printed circuit board; header cores are connected to PCB via nanostructure-based connections; PCB is connected via nanostructure-based connection to feedthrough pins,
- Fig. 6C: shows contacting a cylindrical 4-pole header core by resistance welding an electrically conducting band to the header core wherein the band is configured to provide a nanostructure-based connection to the header core via the resistance welded seams, and
- Fig. 7: shows an embodiment of an implantable device, wherein battery and capacitor are connected to a printed circuit board via nanostructure-based connections.

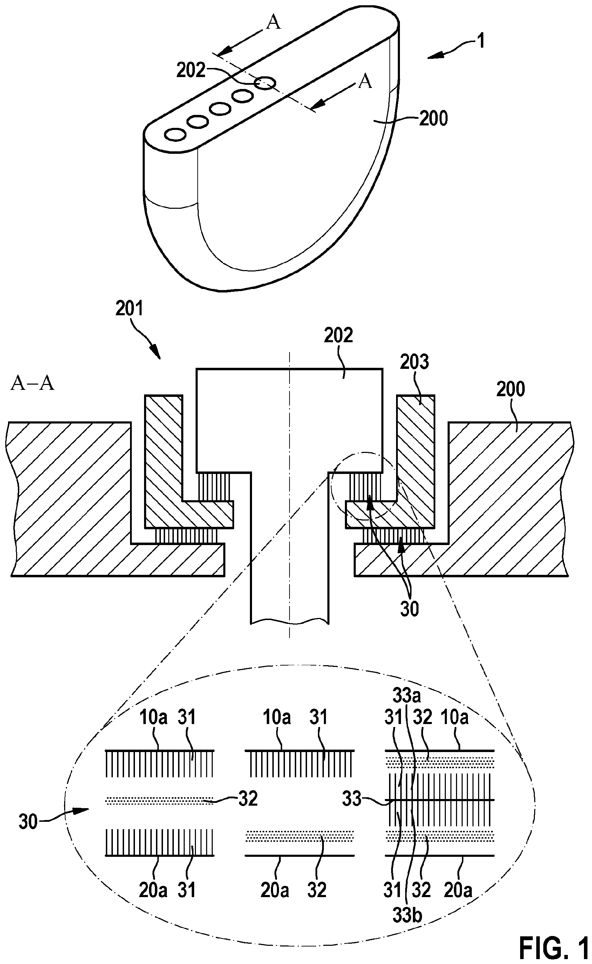

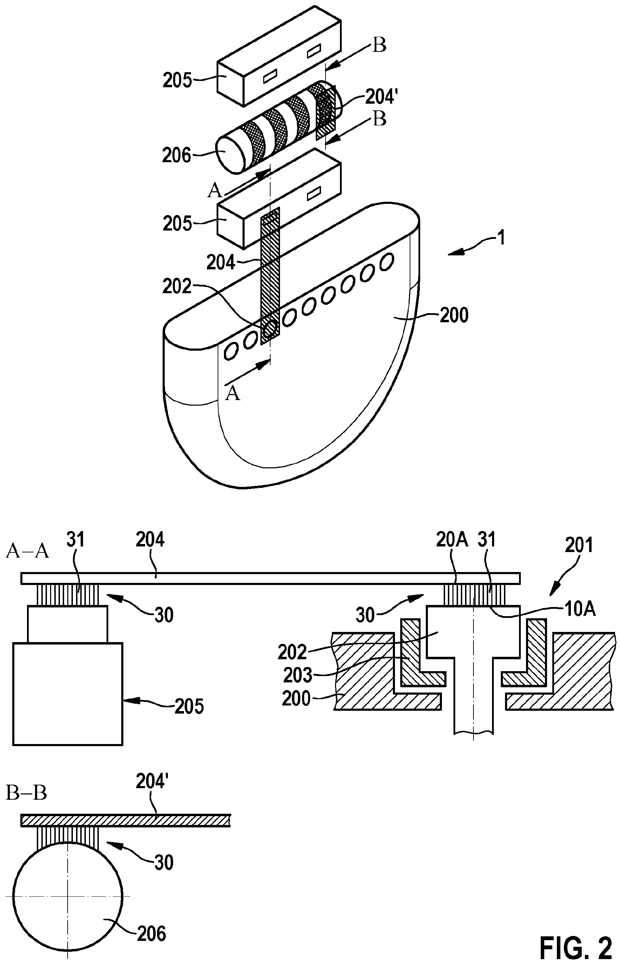

Fig. 1 shows an embodiment of an implantable device 1 (e.g. an implantable pulse generator (IPG) or an implantable cardioverter defibrillator (ICD)) according to the present invention, having a first component, here preferably in form of a feedthrough pin 202 of an electrical feedthrough 201 of a housing 200 of the implantable device 1, wherein the pin 202 is connected to a second component via a connection 30, wherein the second component is a ceramic sleeve 203 that electrically insulates the pin 202 with respect to the housing 200. Here, and in all embodiments described further down below, the connection 30 comprises nanostructures 31 such as nanowires, particularly metallic nanostructures or metallic nanowires 31. These nanostructures 31 can be provided with the individual processes described herein such as Deposition of nanostructures using Direct Laser Interference Patterning (DLIP), Laser Induced Periodic Structures (LIPS), 3D printing, laser cladding, ablation of a material, non-galvanic deposition of a material. Furthermore, as shown in the detail of Fig. 1 - the connection 30 may have different preferred configurations. According to a first preferred embodiment, the pin 202 comprises a surface 10a, that is preferably formed by a circumferential bottom side of a head of the pin, which surface 10a faces a surface 20a of the sleeve 203, wherein both surfaces 10a, 20a can be provided with nanostructures 31 (e.g. as described herein), and pressed onto one another to generate connection 30. Optionally, according to a second embodiment, an adhesive layer 32 may be arranged between the two surfaces 10a, 20a to bond the two surfaces 10a, 20a by means of the adhesive (see above) to one another. According to a third embodiment, the nanostructures 31 may be omitted on one surface, i.e., so that one of the surfaces 10a, 20a is free of nanostructures initially. Also, here, the connection 30 may be made with or without adhesive layer 32. Furthermore, instead of providing nanostructures on the surfaces 10a, 20a, a film 33 may be placed between the surfaces 10a, 20a, wherein now the film 33 is equipped on both sides 33a, 33b of the film 33 with nanostructures 31. The surfaces 10a, 20a are now pressed against each other and connect via the intermediate double-sided nanostructure film 33. Also, here, an adhesive layer 32 may optionally be used between each surface 10a, 20a and the corresponding opposing surface 33a, 33b of the film. Herein, these connections 30 are briefly denoted as nanostructure-based connections 30 or simply as connections 30. Each time a nanostructure-based connection 30 is mentioned herein, this connection may be designed according to one of these embodiments that are also described in detail further above. Also, all connections described herein may be combined in any sensible way. For instance, apart from the nanostructure-based connection 30 between pin 202 and sleeve 203, a further nanostructure-based connection 30 may be provided between a circumferential bottom surface of sleeve 203 and a circumferential opposing surface of housing 200 of the implantable device 1. Particularly, the implantable device 1 comprises a titanium housing 200, wherein preferably the housing 200 comprises five electrical feedthrough pins 202 which may be formed as nail head pins 202 and may be flush with the housing 200. In Fig. 1 all surfaces that are connected to one another via nanostructures 31 are preferably planar surfaces. The connection between the (e.g. titanium) housing 200 (or a flange) and ceramic sleeve 203 and between feedthrough pin 202 and ceramic sleeve 203 are provided to create a hermetically sealed and electrically insulating connection between housing 200 and feedthrough pin 201. The pins 201 establish a hermetically sealed connection through the housing 200 between the electronics inside and the area outside the housing. Fig. 2 shows a further embodiment of an implantable device 1 (e.g. an IPG or ICD) comprising an external wiring to connect a feedthrough pin 202 via an electrically conducting band 204 to a header core (here a two-pole header core) 205. As indicated in the cross-section A-A of Fig. 2, electrically conducting band 204 is connected via a nanostructure-based connection 30 to pin 202 of feedthrough 201, namely to a planar top side of the head of pin 202, and via a further nanostructure-based connection 30 to cuboidal shaped header core 205 which comprises a rectangular cross-section and planar wall for connecting to the band 204.

As before, the nanostructure-based connection 30 may be one of the configurations described herein and further explained in conjunction with Fig. 1 above, i.e. nanostructures 31 are provided on both opposing surfaces of header core 205 and band 204 or only on one of these surfaces. Alternatively, a film 33 as described above may be used and arranged between header core 205 and band 204. Optionally, an adhesive layer may be arranged between each two opposing surfaces, e.g., between header 205 and band 204 or between a film comprising nanostructures and the respective surface of header 205 and band 204 (see also above). As can also be seen in the lowermost cross-section B-B of Fig. 2, due to a certain length of the nanostructures 31 a curvature of the essentially cylindrical 4-pole header core 206 can be compensated for to a certain degree. Here, the nanostructures may be provided on the electrically conducting band 204' and/or on the header core 206, too. Also, here, adhesive layer(s) and/or an intermediate film comprising nanostructures may be used. Based on the nanostructures, an efficient and reliable connection between an electrical feedthrough 201 comprising e.g. nail head feedthrough pins 202 and the contacts of the header cores (2-pole female connectors 205 and 4-pole female connector 206) may be established. The involved components are all located in the header of the implant, i.e. outside the titanium housing 200. Particularly, the area, i.e., the header cores 205, 206 and wiring 204 is encapsulated with resin to form the header. The pacemaker electrodes are configured to be connected to the connector sockets 205, 206, which conduct the electrical signals from ("sense") and to ("pace") the heart. The titanium housing can comprise e.g. 9 feedthrough pins 202 on the lateral circumferential shoulder of the titanium housing 200.

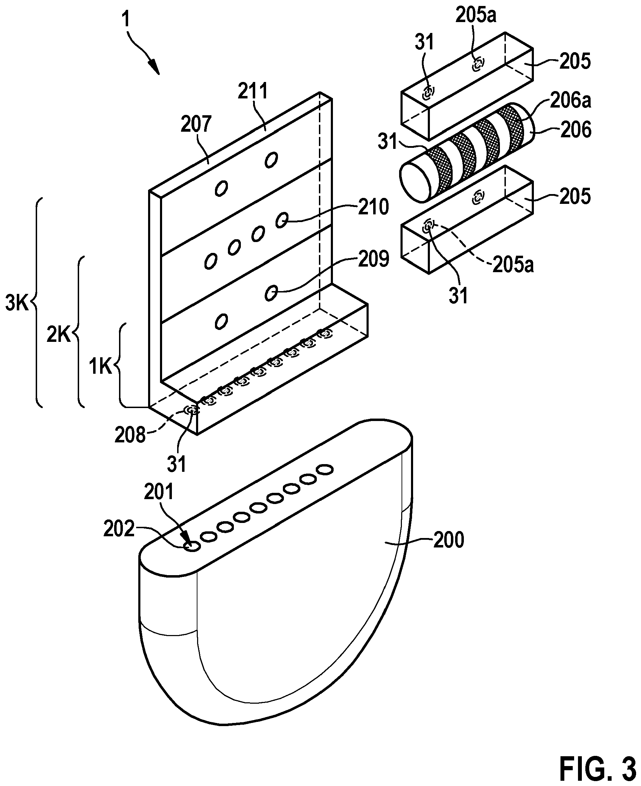

Fig. 3 shows a further embodiment of an implantable device 1 according to the present invention relating to an external wiring of the device 1. Particularly, the implantable device 1 may be an IPG or an ICD, wherein here nanostructure-based connections 30 are made between an electrical feedthrough 201 (e.g. nail head feedthrough pins 202), a printed circuit board or interconnect device 207, particularly a 3D molded interconnect device (MID), and the contacts of the header cores, particularly 2-pole female connector 205 and 4-pole female connector 206. The components are all located in the "header" of the implant, i.e., outside the housing 200; the area, i.e., the header cores 205, 206 and printed circuit board 207, is encapsulated with resin to form the header; the pacemaker electrodes are configured to be connected to the connector sockets 205, 206, which conduct the electrical signals from ("sense") and to ("pace") the heart. Particularly, the housing 200 may be a titanium housing 200 with e.g. nine electrical feedthrough pins 202 on the top of the housing 200, two 2-pole female header cores 205 with two planar contact points, and a 4-pole female connector 206 with four curved contact points. Particularly, the MID 207 comprises contact surfaces 208 that may each connect via a nanostructure-based connection to a surface of an associated pin 202 of the feedthrough 201, wherein nanostructures can be provided on the surfaces of pins 202 and/or on the surfaces of contact points 208 of the MID 207. As described before, optional adhesive layers may be provided between the surfaces of contact points 208 and pins 202. Generally, all nanostructure-based connections described herein may be applied to pins 202 and contacts 208. The headers 205, 206 now connect with their contact point surfaces 205a, 206a to corresponding contact point surfaces 209, 211 and 210 of the MID 207 as shown in Fig. 3, wherein each pair of opposing contact point surfaces 205a, 209, 206, 210, 205a, 211 may comprise nanostructures on both sides or just on one side. Intermediate adhesive layers and films can also be used as described in detail above.

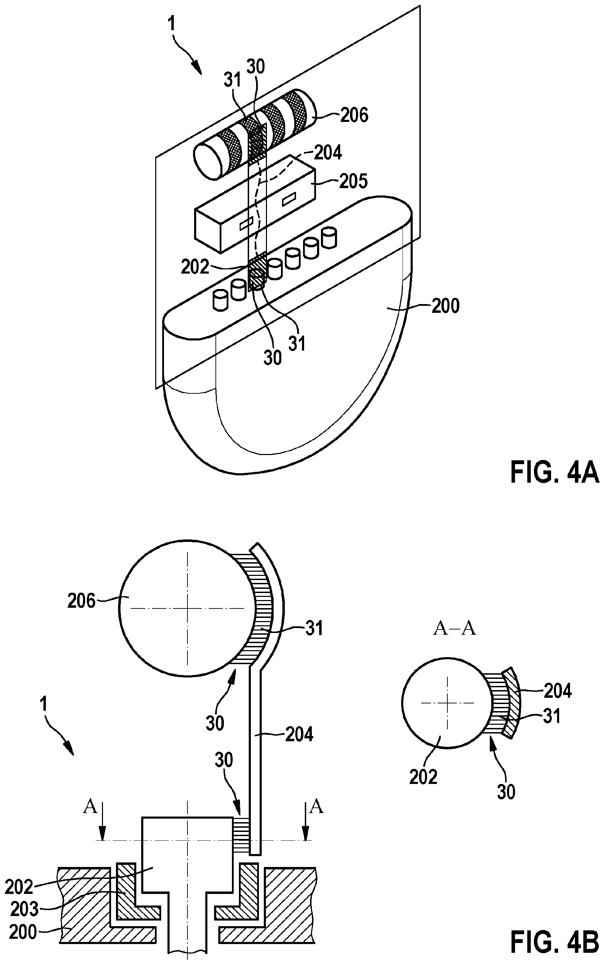

Fig. 4A shows a further embodiment of an implantable device 1 (e.g. an IPG or ICD) according to the present invention. The device 1 comprises an electrically conducting band 204 that provides an electrical connection between a pin 202 of an electrical feedthrough 201 of a housing 200 of the device 1 and a header core 206, here in form of a 4-pole female connector (also 2-pole headers may be connected in this way). Fig. 4B shows a cross-sectional view of the device of Fig. 4A, wherein band 204 is connected laterally to the pin 202 via a nanostructure-based connection 30 that is preferably established according to the embodiments described herein in detail, i.e., both surfaces of the pin 204 and of the band 204 may be provided with nanostructures or merely just one of them. Furthermore, the nanostructures may also be arranged on an intermediary film arranged between the pin 202 and the band 204, wherein the film may nanostructures on each side that can connect to the pin 202 on one side and to the band 204 on the other side. Optionally, an adhesive layer may be provided between each opposing surface involved in the connection as described in detail above (cf. also Fig. 1). Furthermore, as indicated in Fig. 4B, the band 204 is connected to a curved header core 206 (here 4-pole female connector), wherein also here the nanostructures may be provided an both surfaces, i.e., on the header core 206 and on the band 204 or only on one of the two surfaces that shall be connected. In the same manner, a film with nanostructures on both sides may be provided between the two surfaces to be connected instead. Again, optionally, an adhesive layer may be provided between each two opposing surfaces of the connection 30 between header core 206 and band 204 as described above. Particularly, the components involved in the connections 30 are all located in the header of the implant 1, i.e. outside the housing 200; the header cores 205, 206 and the external wiring (band) 204 are encapsulated with resin to form the header; the pacemaker electrodes are configured to be connected to the connector sockets 205, 206, which conduct the electrical signals from ("sense") and to ("pace") the heart. Particularly, the housing 200 may be a titanium housing, and the feedthrough pin 202 can be a nail head pin as nail head pins have the advantage that they have a head and thus a larger joining surface.

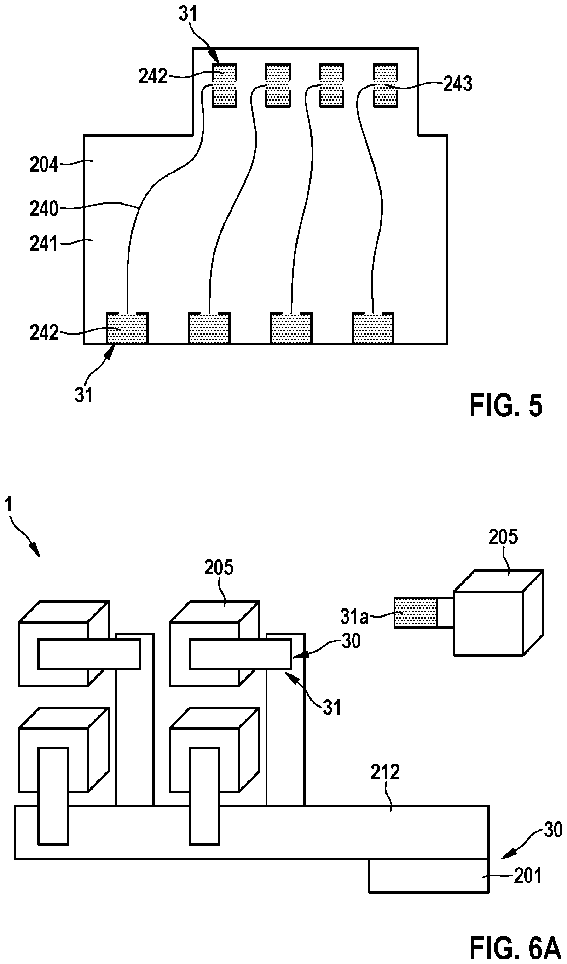

As shown in Fig. 5, generally, electrical connections may be made with a flexible connector 204. The flexible connector 204 may comprise multiple conductive tracks 240 arranged on a flexible substrate 241. Each conductive track 240 connects two opposing contact pads 242 that each comprise nanostructures 31 provided thereon. The flexible connector 204 may therefore be connected to corresponding contacts by pressing the nanostructure-covered pads 242 of the flexible connector 204 against opposing contact surfaces.

The advantage of this design is that the conductor 204 is flat, but due to slots 243 (e.g. cut with laser) formed in the substrate 241 adjacent the nanostructure pads 242, the pads 242 with nanostructures thereon can be easily pressed onto curved surfaces.

Flexible connector as shown in Fig. 5 is therefore particularly suitable to be used as connecting conductor 204 in Figs. 4A, 4B since it can conform to the contour of the header core 206 (e.g. four-pole female connector). However, flexible connector 204 according to Fig. 5 may also be used inside the implant, i.e., in the interior space of the implant enclosed by its housing.

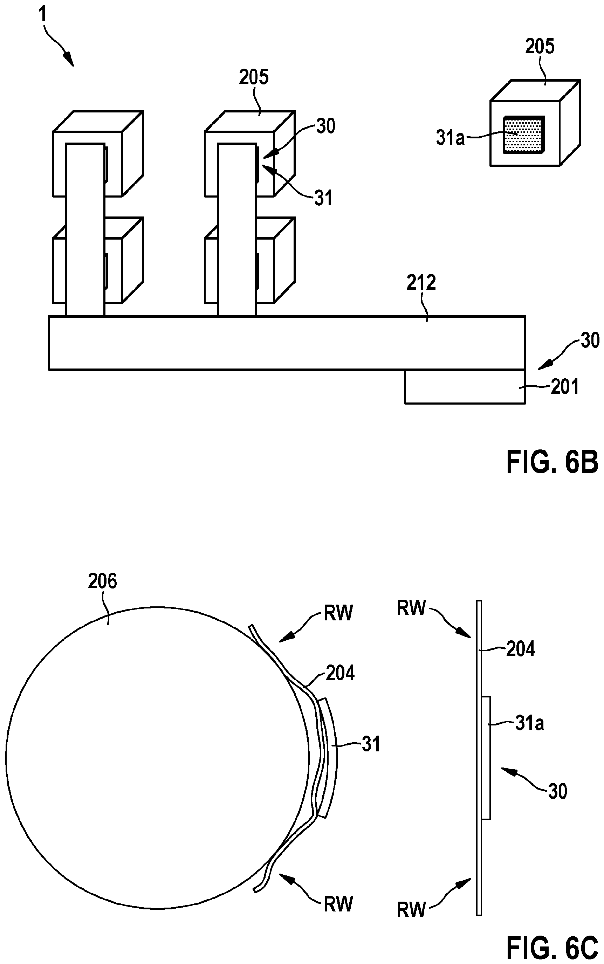

Fig. 6A and 6B show a further embodiment of an implantable device 1 comprising an external wiring to connect header cores 205, here in form of two-pole female connectors 205, to an electrical feedthrough 201 of a housing (not shown) of the device 1. However, the same technique may be used to connect 4-pole connectors to the feedthrough 201. The respective header core 205 can comprise an Au-coating 31a on Ti as substrate without an intermediate Ni layer. This Au-coating can be a nanostructure coating/surface. The header cores 205 may be connected to a printed circuit board (PCB) 212 via nanostructure-based connections 30, and the PCB 212 in turn may be connected to pins of a feedthrough of the housing of the implant 1 via nanostructure-based connections 30, too. However, the PCB 212 may also be connected to the feedthrough 201 without the use of nanostructures 31. Particularly, the pins of the feedthrough 201 may be formed as nail head pins (not shown).

The components are all located in the header of the implant 1, i.e. outside its (e.g. titanium) housing; the area, i.e., the header cores 205 and PCB 212, are encapsulated with resin to form the header. Further, pacemaker electrodes are configured to be connected to the connector sockets 205, which conduct the electrical signals from ("sense") and to ("pace") the heart.

In order to connect the header cores 205 to the PCB 212, the nanostructures may be provided on the pads 31a and/or on the PCB 212 just as described before. Furthermore, also a film may be used for each header core 205 that provides nanostructure surfaces/coatings facing away from one another to connect opposing surfaces, here of the respective header core 205 and the PCB 212. Again, optionally, an adhesive layer may be arranged between opposing surfaces that shall be connected to one another using nanostructures.

As indicated in Fig. 6C, in case a four-pole connector / header core 206 shall be connected to PCB 212, a wiring band / conductor 204 is preferably resistance welded to the header core 206 at to opposing ends, wherein a central region of the conductor 204 comprises nanostructure coating 31a comprising Au nanostructures.

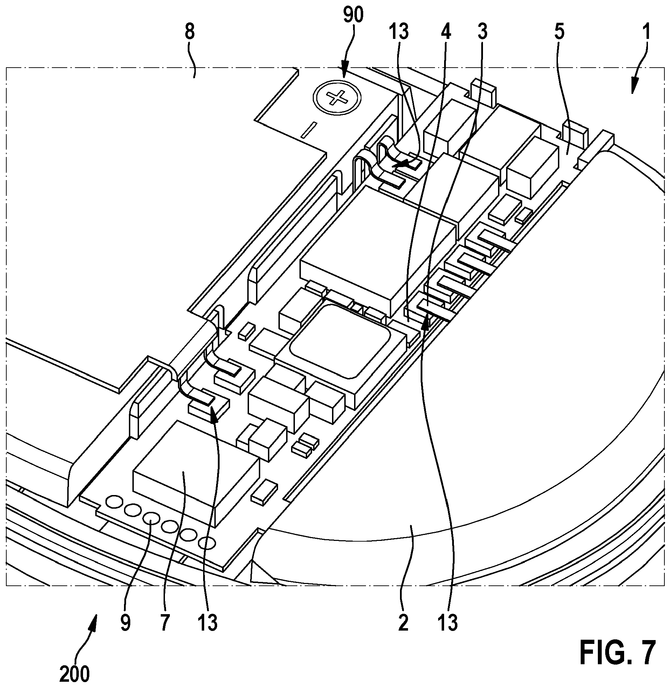

Finally, Fig. 7 shows an application of the present invention to an internal wiring of an implantable device such as an IPG or an ICD. Particularly, nanostructures may be provided on tabs 13, which are preferably CuNi tabs 13. However, other tab materials are also conceivable. With respect to internal wiring virtually everything inside the encapsulated housing 200 can be connected to one another based on nanostructure connections as described herein.

Particularly, a battery 90 of the device 1 may be electrically connected to an electronic module 9 of the device 1 by means of a nanostructure-based connection between tabs 13 of the battery 90 and printed circuit board 5. For this, the nanostructures may be applied to the tabs 13 and the latter pressed on corresponding contacts on the printed circuit board 5 (of the electronic module 9). Thus, the electronic module 9 may be supplied with electrical energy via battery 90, so that the module 9 may provide therapy to the patient by pacing or shocking the heart of the patient.

Furthermore, the electronic module / circuit 9 may be connected to a capacitor 2 in the same fashion by having nanostructures grown on the tabs 13 (e.g. CuNi tabs 13) of the capacitor 2 and pressing the nanostructure tabs 13 against corresponding contacts of the printed circuit board 5. The capacitor 2 allows to generate higher voltages for shocks (in case of an ICD).

Furthermore, the implantable device 1 preferably comprises a dump resistor 8 to convert unneeded shock energy to heat. The dump resistor 8 can also be connected to the PCB 5 and circuit 9 thereon by having nanostructures grown on the tabs 13 (e.g. CuNi tabs 13) of the dump resistor 8 and pressing these tabs 13 against corresponding contacts on the printed circuit board 5.

Regarding the implantable device 1 of Fig. 7 the nanostructures are preferably provided on said tabs 13 however, the respective nanostructure-based connection can be established based on all other configurations of nanostructures, films with nanostructures thereon and optional adhesive layers as described herein.

## Claims

1. A method for connecting a first component and a second component of an implantable device via a connection comprising nanostructures, wherein the nanostructures are provided by means of one of:
- Deposition of nanostructures using Direct Laser Interference Patterning (DLIP),
- Laser Induced Periodic Surface Structures (LIPSS),
- 3D printing,
- laser cladding,
- ablation of a material,
- non-galvanic deposition of a material.

2. The method according to claim 1 or 2, wherein the respective nanostructure comprises a length in the range from 100nm to 100 µm, and/or a diameter in the range from 10 nm to equal to or below 1 µm.

3. The method according to one of the preceding claims, wherein the respective nanostructure is an elongated nanowire.

4. The method according to one of the preceding claims, wherein the respective nanostructure comprises at least one branching point at which the nanostructure branches out from a base into at least two sections.

5. The method according to one of the preceding claims, wherein the respective nanostructure comprises a free end section forming a hook.

6. The method according to one of the preceding claims, wherein first nanostructures (31) are provided on a surface (10a) of the first component and second nanostructures (31) are provided on a surface (20a) of the second component, wherein the first and the second nanostructures (31) are interlocked with each other.

7. The method according to one of the claims 1 to 5, wherein the nanostructures (31) are provided on a surface (10a) of the first component, wherein the nanostructures (31) and a surface (20a) of the second component (20) are bonded to one another [to form said connection], or wherein the nanostructures (31) are provided on a surface (20a) of the second component, wherein the nanostructures (31) and the surface (10a) of the first component are bonded to one another.

8. The method according to one of the claims 1 to 5, wherein the connection (30) comprises an intermediate film (33) having a first side (33a) and a second side (33b) facing away from the first side (33a), wherein the nanostructures (31) are provided on the first and on the second side (33a, 33b), wherein the nanostructures (31) provided on the first side (33a) are bonded to a surface (10a) of the first component (10), and wherein the nanostructures (31) provided on the second side (33b) are bonded to a surface (20a) of the second component (20).

9. The method according to one of the claims 1 to 5, wherein the nanostructures (31) are provided on a surface (10a) of the first component and/or on a surface (20a) of the second component, wherein the two opposing surfaces (10a, 20a) are connected to one another by an intermediate adhesive layer (32).

10. The method according to one of the claims 1 to 5, wherein the connection (30) comprises an intermediate film (33) having a first side (33a) and a second side (33b) facing away from the first side (33a), wherein the nanostructures (31) are provided on the first and the second side (33a, 33b), wherein the nanostructures (31) provided on the first side (33a) are bonded to a surface (10a) of the first component via an adhesive layer (32), and wherein the nanostructures (31) grown on the second side (33b) are bonded to a surface (20a) of the second component via an adhesive layer (32).

11. The method according to one of the claims claim 6 to 10, wherein the nanostructures are provided on the respective surface (10a, 20a) or side (33a, 33b) so as to form a plurality of spaced apart regions, each region comprising a plurality of nanostructures.

12. The method according to one of the preceding claims, wherein
- the first component is a housing of the implantable device, and wherein the second component is an X-ray marker that is bonded to the housing via said connection, or wherein
- the first component is a shell of a housing of the implantable device, particularly a half shell, and wherein the second component is a shell of the housing, particularly a half shell, wherein said connection closes the shells of the housing hermetically.

13. The method according to one of the claims 1 to 11, wherein the implantable device (1) comprises one of:
- an energy storage, particularly a capacitor (2) comprising an electrical feedthrough pin (3) forming the first component and a circuit (4) arranged on a substrate (5), the circuit (4) forming the second component,
- a printed circuit board (5) forming the first component, the surface (10a) of the first component (5) forming an electrical contact of the printed circuit board, and wherein the second component (7) is an electronic component.
- comprises a dump resistor (8) forming the first component and a printed circuit board (5) forming the second component.

14. The method device according to one of the claims 1 to 11, wherein the implantable device (1) comprises a housing (200) comprising an electrical feedthrough (201) comprising at least one pin (202) for providing an electrical connection to a header (300) connected to the housing (200), wherein the at least one pin (202) is electrically connected to an electronic module (9) enclosed by the housing (200) of the implantable device (1), and wherein
- the at least one pin (202) forms the first component, and the second component is an electrically insulating sleeve (203) that surrounds the at least one pin (202) and is connected to the housing (200), or wherein
- the at least one pin (202) is surrounded by an electrically insulating sleeve (203) that forms the first component, and the second component is formed by the housing (200), or wherein
- the at least one pin (202) forms the first component, and the second component is arranged in the header (300), wherein particularly the second component is an external wiring (204) arranged outside the housing (200) for connecting the at least one pin (202) to a component of the header (300), or wherein
- the first component is formed by an external wiring (204) connected to the at least one feedthrough pin (202), and the second component is formed by a header core (205, 206) of the header, wherein particularly the header core (205, 206) is one of: a socket, a two-pole socket, a four-pole socket, or wherein
- the first component is formed by a first header core of the header and the second component is formed by a second header core of the header, or wherein
- the at least one pin (202) forms the first component, and the second component is an interconnect device (207), particularly a 3D molded interconnect device (MID), that is particularly configured to connect the at least one pin (202) to a header core (205, 206), or wherein
- the first component is an interconnect device (207), particularly a 3D molded interconnect device (MID), that is particularly configured to connect the at least one pin (202) to a header core (205, 206), and wherein the second component is a header core (205, 206) of the header (300).

15. An implantable device (1), comprising
- a first component (3, 5, 8, 202, 203, 204, 207), and
- a second component (4, 7, 5, 203, 200, 204, 205, 206, 207),
wherein
the first and the second component (3, 4; 5, 7; 8, 5; 202, 203; 203, 200; 202, 204; 204, 205, 206; 202, 207; 207, 205, 206) are joined to one another via a connection (30) comprising nanostructures (31) provided by one of:
- Deposition of nanostructures using Direct Laser Interference Patterning (DLIP),
- Laser Induced Periodic Surface Structures (LIPSS),
- 3D printing,
- laser cladding,
- ablation of a material,
- non-galvanic deposition of a material.
